Europäisches Patentamt

(19) European Patent Office

Office européen des brevets

(11) Publication number: **0 150 897**
**A2**

(12) **EUROPEAN PATENT APPLICATION**

(21) Application number: **85300038.8**

(22) Date of filing: **03.01.85**

(51) Int. Cl.⁴: **B 01 J 21/06,** B 01 J 29/02, C 07 C 1/04, C 07 C 29/15

(30) Priority: **06.01.84 GB 8400271**

(43) Date of publication of application: **07.08.85**
**Bulletin 85/32**

(84) Designated Contracting States: **BE DE FR GB IT NL**

(71) Applicant: **The British Petroleum Company p.l.c.,**
**Britannic House Moor Lane, London EC2Y 9BU (GB)**

(72) Inventor: **Atkins, Martin Philip, The British Petroleum Co.**
**p.l.c. Chertsey Road, Sunbury-on-Thames Middlesex,**
**TW16 7LN (GB)**

(74) Representative: **Harry, John et al, BP INTERNATIONAL**
**LIMITED Patents Division Chertsey Road,**
**Sunbury-on-Thames Middlesex TW16 7LN (GB)**

(54) **Novel layered clays, process for their production and uses thereof.**

㊳ The invention provides a silanised layered clay comprising a layered clay chemically modified by incorporation therein of a silicon-containing residue in the absence of a polymeric cationic hydroxy inorganic metal complex. The silanised layered clays are produced by hydrolysing a hydrolysable silicon compound, for example a tetraalkoxysilane, in the presence of the layered clay and in the absence of a polymeric cationic hydroxy inorganic metal complex. The resulting silanised layered clays are more thermally and/or hydrothermally stable than the layered clays from which they are derived.

1

NOVEL LAYERED CLAYS, A PROCESS FOR THEIR PRODUCTION
AND USES THEREOF

The present invention relates to novel layered clays, a process for their production and to uses thereof.

Natural and synthetic clays having a lamellar structure with interlamellar spaces disposed between the lamellar layers are well known. Smectites, such as bentonite and montmorillonite, are a class of clays possessing such a lamellar structure. Montmorillonite has an idealised stoichiometric composition corresponding to $Na_{0.67}[Al_{3.33}Mg_{0.67}](Si_8)O_{20}(OH)_4$. Structurally it comprises a central layer containing octahedrally coordinated aluminium and magnesium in the form of their oxides and hydroxides sandwiched between two layers containing tetrahedrally coordinated silicon essentially in the form of its oxide. Normally in nature cations are present to compensate for the charge imbalance caused by isomorphous substitution of $Mg^{2+}$ for $Al^{3+}$ in the octahedral layer, and/or $Al^{3+}$ or other ions for $Si^{4+}$ in the tetrahedral layers. The octahedral and tetrahedral regions are tightly bound together to form a lamellar layer. The space between these lamellar layers, i.e. the interlamellar space, in natural clays is normally occupied by exchangeable $Ca^{2+}$ or $Na^+$ ions. The distance between the interlamellar layers can be increased substantially by adsorption of various polar molecules such as water, ethylene glycol, amines etc, which enter the interlamellar space and in doing so push the layers apart. It is generally recognised that such clays when heated in air suffer irreversible collapse of their layered structures at temperatures of

1

180°C and above, the actual value of this temperature depending upon the nature of the exchangeable cation present in the interlamellar region of the structure. The collapse of the layered structure is associated with a loss in the catalytic activity of the clay. Layered clays can also suffer from the disadvantage that they suffer particle/granule instability in the presence of polar liquids such as methanol or ethanol. Generally, this does not impair catalytic activity in batch or continuous reactions in which the catalyst particles are subject to agitation, thereby maintaining them in suspension. However, the catalyst particles have at some stage to be separated from the reactor product by filtration, centrifugation or other solid/liquid separation methods. For fixed bed operation, the layered clay catalyst is preferably mixed with suitable binders to ensure granule or pellet stability.

Also known from U.S. Patents Nos. 4176090; 4271043 and 4248739 are stabilised pillared layered clays in which the layers are separated and supported by "pillars" of monomeric, oligomeric or polymeric species.

USP 4,271,043 describes and claims a process for preparing a pillared interlayered clay product having a high degree of ion exchange capacity which comprises:

(a)     reacting a smectite clay with a mixture of a polymeric cationic hydroxy metal complex selected from the group consisting of polymeric cationic hydroxy aluminium and zirconium complexes and water to obtain a pillared interlayered smectite;

(b)     calcining said interlayered smectite to obtain an interlayered clay product having greater than 50% of its surface area in pores less than 30 Angstroms in diameter; and

(c)     reacting said calcined interlayered clay product with a base to increase the ion exchange capacity thereof.

USP 4,248,739 describes and claims a method for preparing a pillared interlayered smectite clay product wherein a smectite clay is reacted with a mixture of polymeric cationic hydroxy metal complex and water to obtain a pillared, interlayered smectite having greater than 50 percent of its surface area in pores of less than 30 Angstroms in diameter after dehydration wherein the improvement comprises reacting said smectite with a high molecular weight cationic hydroxy metal complex and copolymers thereof having a molecular weight of from about 2,000 to 20,000.

Both USP 4,271,043 and USP 4,248,739 describe the use of high molecular weight polymers which may be prepared by copolymerising an aluminium or zirconium metal complex with a copolymerising reactant which may be included in the reaction mixture as sodium silicate, zirconium chloride, boric acid or sodium borate. There is no disclosure of the use of sodium silicate other than as a component of a high molecular weight polymer, as used in "pillaring" the layered clay. The pillared layered clays so produced are said to be useful as sorbents, catalysts and catalyst supports. It is claimed that the pillaring treatment, irrespective of the nature of the pillars, improves the hydrothermal stability of the catalyst to regeneration cycles in cracking reactions.

We have now found that both the hydrothermal and thermal stability of layered clays can be improved without the introduction of the polymeric cationic hydroxy inorganic metal complexes referred to hereinbefore.

Accordingly, the present invention provides a silanised layered clay comprising a layered clay chemically modified by incorporation therein of a silicon-containing residue in the absence of a polymeric cationic hydroxy inorganic metal complex.

According to another aspect of the present invention there is provided a process for the production of a silanised layered clay which process comprises hydrolysing in the presence of a layered clay a hydrolysable silicon compound in the absence of a polymeric cationic hydroxy inorganic metal complex.

The silanised clays differ from the layered clays from which they are derived in the respects that they contain more silicon and they have greater hydrothermal and thermal stability, thereby allowing their use under a wider range of conditions.

The layered clay used as starting material in the process of the invention may be a natural or a synthetic layered clay. Of the many types of layered clay available (see for Example "Encyclopedia of Chemical Technology [Kirk Othmer], 3rd Edition, published by John Wiley and Sons, pages 190-206), it is preferred to use the smectite class of layered clays. Both dioctahedral and trioctahedral smectite-type clays may be employed. For an explanation as to the difference between dioctahedral and trioctahedral-type clays reference is made to the book entitled "The Chemistry of Clay – Organic Reactions" by B.K.G. Theng, published by John Wiley and Sons, New York-Toronto, at page 2. Examples of suitable layered clays of the smectite class include montmorillonite, bentonite, beidellite, nontronite and hectorite. An example of a suitable synthetic smectite – type layered clay and a method for its preparation is described in US Patent No. 3855147.

Any suitable hydrolysable silicon compound may be employed in the method of the invention. Suitable hydrolysable silicon compounds include silicon halides and, more conveniently, the tetraalkoxysilanes, wherein the alkoxy groups may be the same or different. An example of a tetraalkoxysilane which may be used in the method of the invention is tetraethoxysilane. The amount of the hydrolysable silicon compound to be employed will depend upon the amount of silicon it is desired to incorporate into the layered clay.

Hydrolysis of the hydrolysable silicon compound may suitably be achieved by contacting the compound in the liquid phase with a hydrolysing agent in the presence of the layered clay under conditions which do not impair the layered structure of the clay, preferably at a temperature above ambient, but at a temperature at which the layered structure of the clay is unimpaired. Suitably the temperature may be less than 100°C. The hydrolysing agent may suitably be water, aqueous alcohol, dilute aqueous alkali or dilute aqueous acid, and is preferably water. The hydrolysis mixture should be agitated during the hydrolysis in order to maintain a uniform dispersion. Hydrolysis may be effected over a period of from a few minutes to several days, preferably from about 30 minutes to about 6 hours.

The so-treated layered clay may thereafter be separated from the hydrolysate by conventional means, such as by centrifugation or filtration. It may thereafter be dried.

Alternatively, the layered clay may be contacted with the hydrolysable silicon compound in the vapour phase under conditions which do not impair the layered structure of the clay. For this method of operation the presence of a hydrolysing agent is unnecessary.

It is preferred in a final step to heat the treated layered clay to an elevated temperature such that substantial collapse of the layered structure is avoided, which temperature may be as high as about 400°C depending upon the nature of the layered clay, the exchanged cation and the amount of silicon-containing residue therein, preferably at about 250 to 350°C for a period of, for example from $\frac{1}{2}$ to 12 hours. The heating may suitably be carried out in air, oxygen or an inert gas, such as nitrogen.

The layered clay having an increased silicon content may be used as a catalyst or a catalyst support in a wide variety of chemical reactions. For such use it will usually be beneficial either to exchange the cations normally associated with the layered clay with other cations, for example hydrogen ions and/or metal cations, and/or to impregnate the layered clay with acids and/or metal compounds. Ion-exchange of the layered clay, may suitably be effected either before or after hydrolysis using techniques known in the art. Impregnation of the layered clay may suitably be effected after hydrolysis, again using techniques known in the art. Reactions in which layered clays obtained by the method of the invention may be used as catalysts include the conversion of synthesis gas to hydrocarbons and/or alcohols and reactions which are catalysed by protons, such as for example esterification, etherification, hydration and alkylation.

Operating conditions for the conversion of synthesis gas to hydrocarbons and/or alcohols are well known in the art and require no further elaboration. The use of layered clays as catalysts in certain reactions which are catalysed by protons are described for example in our copending European applications publication Nos. 31252, 31687, 45618 and 110628 to which the reader is referred for further details of reactants and reaction conditions which may be employed.

The method of the invention will now be further illustrated by reference to the following Examples.

SILANISATION OF A MONTMORILLONITE (SMECTITE-TYPE LAYERED CLAY)

Example 1

Tetraethyl orthosilicate (35.6g) was added to a 250ml round bottom flask containing the hydrogen form of montmorillonite (Wyoming) clay. The mixture was stirred rapidly for 5 minutes at room temperature. To the stirred slurry was added deionised water ($71cm^3$). The contents of the flask were heated at 70°C for 1 hour and thereafter the treated clay was separated and subsequently dried in an oven at 100°C.

The silanised product had a silicon to aluminium ratio of 4.9:1 as determined by XRF analysis. The original montmorillonite clay had a silicon to aluminium ratio of 2.3:1.

The silanised layered clay had an intact basal $d_{001}$ spacing of 11.5 Angstrom as determined by XRD. On heating in air overnight up to 325°C the basal $d_{001}$ spacing remained at 11.5 Angstrom whereas the hydrogen form of the montmorillonite used as the starting material collapsed upon heating to 275°C under the same conditions. At 350°C the silanised layered clay collapsed to a $d_{001}$ of 9.6 Angstroms.

Granules of the silanised layered clay were slurried separately with water and methanol as representative polar solvents. No evidence of instability was detected. This behaviour is to be compared with that of the hydrogen form of the montmorillonite clay used to prepare the silanised clay, which clay "sanded" instantaneously on contact with water.

Example 2

The procedure of Example 1 was repeated using an aluminium-exchanged montmorillonite (Texas) layered clay.

Granules of the silanised layered clay so-produced were slurried separately with water and methanol as representative polar solvents. No evidence of instability was detected.

CATALYTIC ACTIVITY OF SILANISED LAYERED CLAY

Example 3 - The Production of Ethyl Benzene

Benzene (120g) and a portion (5g) of the silanised layered clay obtained in Example 1 were charged to a 250ml autoclave. The autoclave was charged with ethylene (to 40 bars) and the contents of the autoclave were heated at 200°C for $2\frac{1}{2}$ hours.

The liquid products were analysed by gas chromatography and found to contain 13% wt ethyl benzene.

Example 4 - The Production of Methyl Tertiary-Butyl Ether (MTBE)

A portion (10 cm$^3$) of the silanised layered clay obtained in Example 1 was charged to a small continuous reactor. To the reactor was fed:

|            | % MOLAR |
|------------|---------|
| MeOH       | 10      |
| isobutene  | 10      |
| n-butenes  | 10      |
| pentane    | 70      |

The reactor was maintained at 80°C and 30 bar pressure with a residence time of 15 minutes.

The products were analysed by gas chromatography and comprised:

|                     | % WT |
|---------------------|------|
| MeOH                | 14.3 |
| n- plus iso-butenes | 63.8 |
| MTBE                | 21.9 |

Example 5 - The Production of MTBE

The method of Example 4 was repeated using a portion of the silanised layered clay obtained in Example 1 which had been exchanged with hydrogen ions.

34.8% weight MTBE was found in the product stream.

This Example demonstrates that the catalytic activity of the silanised layered clay can be markedly increased by acid treatment.

Claims:

1. A silanised layered clay comprising a layered clay chemically modified by incorporation therein of a silicon-containing residue in the absence of a polymeric cationic hydroxy inorganic metal complex.

2. A process for the production of a silanised layered clay which process comprises hydrolysing in the presence of a layered clay a hydrolysable silicon compound in the absence of a polymeric cationic hydroxy inorganic metal complex.

3. A process according to claim 2 wherein the layered clay is a smectite.

4. A process according to either claim 2 or claim 3 wherein the hydrolysable silicon compound is a tetraalkoxysilane, wherein the alkoxy groups are either the same or different.

5. A process according to claim 4 wherein the tetraalkoxysilane is tetraethoxysilane.

6. A process according to any one of claims 2 to 5 wherein hydrolysis of the hydrolysable silicon compound is achieved by contacting the compound in the liquid phase with a hydrolysing agent in the presence of the layered clay under conditions which do not impair the layered structure of the clay.

7. A process according to any one of claims 2 to 5 wherein hydrolysis of the hydrolysable silicon compound is achieved by contacting the compound in the vapour phase with the layered clay under conditions which do not impair the layered structure of the clay.

8. A process according to any one of claims 2 to 7 wherein the silanised layered clay is heated to a temperature in the range from 250 to 350°C.

8

9. A silanised layered clay when used as a catalyst in reactions which are catalysed by protons.

10. A silanised layered clay when used as a catalyst in a process for the conversion of synthesis gas to hydrocarbons and/or alcohols.